# EUROPEAN PATENT SPECIFICATION

(11) **EP 0 843 739 B1**
(45) Date of publication and mention of the grant of the patent: **07.11.2007**
(21) Application number: 96927398.6
(22) Date of filing: 09.08.1996
(51) Int. Cl.: C12Q 1/68, C12Q 1/70, C12P 19/34, G01N 33/53, C07H 21/04, G01N 33/543, C07H 21/00

(54) **MOLECULAR COMBING OF MACROMOLECULES THROUGH THE ACTION OF A MENISCUS ON HIHGLY SPECIFIC SURFACES**
MOLEKULARE AUSRICHTUNG VON MAKROMOLEKUELEN MIT HILFE EINES FLUESSIGKEITSMENISKUS AUF EINER HOCHSPEZIFISCHEN OBERFLAECHE
PROCEDE MOLECULAIRE POUR PEIGNER DES MACROMOLECULES PAR L'ACTION D'UN MENISQUE SUR DES SURFACES HAUTEMENT SPECIFIQUES

(30) Priority: 09.08.1995 US 512980
(43) Date of publication of application: 27.05.1998
(73) Proprietor: Simon, Adam, J., Yardley, PA 19067 (US)
(72) Inventor: Simon, Adam, J., Yardley, PA 19067 (US)
(74) Representative: Lerner, François
(86) International application number: PCT/US1996/013081
(87) International publication number: WO 1997/006278

(56) References cited:
- EP-A1- 0 305 407
- EP-A2- 0 578 148
- WO-A1-92/08788
- WO-A1-95/21939
- WO-A1-95/22056
- US-A- 4 562 157
- US-A- 5 102 798
- US-A- 5 511 428
- BENSIMON A ET AL: "ALIGNMENT AND SENSITIVE DETECTION OF DNA BY A MOVING INTERFACE" SCIENCE, AMERICAN ASSOCIATION FOR THE ADVANCEMENT OF SCIENCE,, US, vol. 265, 30 September 1994 (1994-09-30), pages 2096-2098, XP002036061 ISSN: 0036-8075
- WEIER H -U G ET AL: "Quantitative DNA fiber mapping." HUMAN MOLECULAR GENETICS, vol. 4, no. 10, 1995, pages 1903-1910, XP009008685 ISSN: 0964-6906
- BENSIMON D. ET AL.: 'Stretching DNA with a Receding Meniscus: Experiments and Models' PHYSICAL REVIEW LETTERS vol. 74, no. 23, 05 June 1995, pages 4754 - 4757, XP001064957

## Description

### BACKGROUND OF THE INVENTION

### Field of the Invention

The present invention relates to highly specific surface (s) that may be used in biology, and to substrates, constructs, and devices utilizing the surface (s), as well as their applications and processes for their preparation. The invention also relates to a method for stretching macromolecules such as polymers or biologically active macromolecules, particularly DNA, or proteins.

### Background of the Invention

The high specificity and selectivity of certain biologically active reactions has been known for a long time. Specifically, the antibody-antigen reactions, the DNA or RNA hybridizations, the reactions between proteins of the type avidin/streptavidin-biotin as well as the reactions between ligands and their receptors are well known. It is now possible to take advantage of these specific reactions, particularly to demonstrate the presence or absence of one of the elements of the reaction coupled in a sample, or eventually to separate one of the elements of the couple from a more complex media. However, when it is desired to detect the presence of a molecule at very low concentrations, in a complex media, the currently available processes often provide random results, especially because of background noise experienced during the separation or detection steps. This is why what will be called hereinafter "molecular fishing", that is the possibility to detect each of the molecules which are sought after even at very low concentrations, has not yet been possible. For example, the analysis of a DNA sample requires the use of a hybridization probe corresponding to the complementary sequence of the sequence to identify. In these conditions, the problem is to isolate the hybrid from the medium, then to detect it with a good signal to noise ratio (SNR), and eventually determine even a small number of positive reactions. This is why in many cases, an intermediate step to amplify the sequence to detect, for example by using PCR or amplification methods leading to the same results, is used. In these conditions, the concentration of the sequence to analyze in the sample is increased and its detection is rendered much easier. However, this amplification step is sensitive to contaminants which eventually lead to detection errors. It would therefore be desirable to be able to detect the presence of the nucleic acid sequence with no amplification step. It has been proposed to use, in order to highlight the hybridization reaction, an intermediate anchoring step of the hybridization product onto a solid surface presenting certain binding specificities. For example, it is possible to use certain pretreated surfaces which allow binding certain proteins or DNA, whether this DNA has been modified or not. Such surfaces are commercially available (Covalink^{R}, CoStar^{R}, Estapor^{R}, Bangs^{R} and Dynal^{R} for example) in the form of beads or wells having on the surface - COOH, -NH₂ or -OH groups for example. The DNA can be therefore functionalized with a reactive group, an amine for example, and the reaction can proceed with these surfaces. However, these methods require a particular pretreatment or functionalization of the DNA to be bound.

Techniques allowing the anchoring without pretreatment of the DNA have also been described. One of those techniques consists in reacting the free phosphate group of the 5' end of the molecule with a secondary amine. It is also possible to fix the DNA to a group of a protein in order to react it with a surface covered with a group of a second protein susceptible of specifically reacting with the first protein. The bound complex (first protein-second protein) can be of the type biotin-streptavidin, and-digoxygene antibody (anti-DIG) directed against digoxygene antigen (DIG). Such surfaces are however in most cases not sufficiently specific. Thus, the presence of parasitic interactions, even weak ones, of the type non-specific adsorption, can lead, for a long molecule, to absorption on the solid at a large number of weak interaction points (V. Lund et al., Nucl. Acids Res. 16, 1861 (1988)). These surfaces lead to potential applications lacking sensitivity and/or with a high rate of background noise in the case of a small number of molecules to "fish." Furthermore, some of these surfaces present a high rate of parasitic fluorescence which is potentially detrimental during the detection phase (if photons are used in detection). As for the detection itself, in particular for highlighting the presence of DNA, French Patent 78 10975 describes a process coupling a probe to an enzyme which allows one to observe the release of a chromatic substrate, that is a dye which when released changes its optical properties (either emission or absorption characteristics). It is furthermore possible to quantify the reaction by an optical density, spectral or calorimetric measurement. A technique of this type is, however, not directly adapted for the detection of trace quantities. Therefore, this technique must also be preceded, in most cases, by an amplification process of the quantity of nucleic acids sought after, for example by the PCR method. The detection process described as the "cold probe technique" has been developed to avoid the use of radioactive markers, which give results which are close in sensitivity but present manipulation problems, taking into account the presence of radioactivity and the long development times necessary for good sensitivity. For certain particular applications, such as methods devised from *ex-vivo* imaging, direct methods of observation of the reaction have been proposed by coupling the hybridization product to microbeads, particularly to PMMA, conveniently treated chemically on their surface. The method relies on the direct identification, under an electron microscope, of the presence of these microbeads (of a typical diameter of 60 nanometers) and also relies on known solid anchoring techniques that are not sufficiently specific.

The techniques described above are obviously not limited to the detection of nucleic acids. The detection of antibodies using the same systems have been suggested. In these situations, the tests are ELISA type tests, which will not be described here, and which, to summarize, allow the coupling of an antibody to an anchor site associated with an antigen molecule on a solid. In this situation, the specificity and parasitic reaction problems are still present. The detection method can be based on a coupling to a chromatic substrate, which has its own sensitivity problems.

### SUMMARY OF THE INVENTION

This invention relates to a reproducible method of combing, orienting, aligning, straightening or stretching a macromolecule on a surface, which comprises placing a macromolecule in contact with a surface S and a medium A, so that one site of the macromolecule is bound to S and a portion thereof is placed in A, and mechanically translating the contents of A relative to S, under conditions effective to comb, orient, align, straighten or stretch the macromolecule.

The present invention also relates to a method of obtaining a surface S affording consistent, homogeneous, and reproducible orientation, alignment, straightening or stretching of macromolecules, which comprises cleaning a surface S of a substrate under conditions effective to obtain an organic molecule-free and dust-free surface S, hydrating S, and coating S with a desired material comprising exposed moieties which are capable of selectively binding macromolecules; the cleaning, hydrating and coating steps being conducted in a single chamber.

Also part of this invention is a surface prepared by the method described above, suitable for use in the orientation, alignment, straightening or stretching of macromolecules, in a consistent, homogeneous, and reproducible manner, also provided in this patent are a substrate, construct or device comprising the surface of the invention, and kits comprising the surface, substrate, construct and/or device, and other components, and instructions for its use, suitable for various applications described below.

The surface of this invention is advantageously applied attaining a desired degree of orientation, alignment, straightening or stretching of a macromolecule bound to a surface S, by placing the macromolecule in contact with the surface of the invention and a medium A, so that one site of the macromolecule is bound to the surface and a portion thereof is placed in A, modifying the hydrophobic/ hydrophilic characteristic of either the surface or A, in a manner effective to produce a desired degree of stretching of the macromolecule, evaporating A or translating the contents of A relative to the surface to orient, align, straighten, or stretch the macromolecule, and determining the length of the stretched macromolecule by comparing to a standard or control.

Document WO-A-92/08788 discloses a coated substrate having the ability to bind macromolecules in an oriented position, and which is cleaned in order to remove any organic molecule and dust from it, then hydrated and coated with a reagent. The substrate may be obtained after UV irradiation. Macromolecules may be oriented by a doctor blade technique. It does not disclose a method for obtaining a surface including steps which are all executed in a single chamber, nor a translating step in which two surfaces are moved parallely for translating an interface.

The invention relates to a method for obtaining a surface S affording consistent, homogeneous and reproducible orientation, alignment, straightening or stretching of macromolecules according to claim 1, or according dependent claim 2. The invention also relates to a reproducible method of orienting, aligning, straightening or stretching a macromolecule on a surface, according independent claim 4, or according dependent claim 3 or 5 to 9. The invention also relates to a surface S obtained by a preceding method.

Another application of the surface of the invention is, for example, in a sensitive method of identifying a specific type of macromolecule present in a sample, by contacting the macromolecule with the surface and the medium, and orienting the macromolecule as described above, and detecting the presence of the stretched macromolecule in a quantitative and reproducible manner, and specifically identifying the type of the oriented macromolecule.

Still another application is in the determination of the number of a specific-type of macromolecule present in a known volume of sample, by contacting a macromolecule with the surface and the medium, orienting, the macromolecule, and detecting the presence of the oriented macromolecule as described above, and determining the number of oriented macromolecules present in a reproducible and quantitative manner.

Still another use of the surface is in the separation of a specific-type from other types of macromolecules by contacting a macromolecule with the surface and the medium A, evaporating A or translating the contents of A relative to the surface to displace A, and other sample components while the specific-type macromolecule (s) remain bound to the surface and is (are) oriented, and detecting the presence of any oriented specific-type macromolecule (s) in a reproducible and quantitative manner.

Yet another application of the surface of the invention is in the measuring the length of a specific-type macromolecule by placing a macromolecule having a marker at a position of known length in contact with the surface of the invention and a medium A, evaporating A or translating its contents relative to the surface while the macromolecule remains bound to the surface to orient the macromolecule, and determining the length of any fragment of the oriented macromolecule extending between the molecule and the macromolecule's portion placed in A by comparison to a standard or control.

The surface of the invention is also suitable for measuring a desired distance between two points on a surface by applying the above steps, and further determining the desired distance by comparison to a known length.

### BRIEF DESCRIPTION OF THE DRAWINGS

Subfigure 1A is a schematic representation of the detection of a pathogen in a fluorescent DNA molecule by hybridization with an anchoring molecule. Subfigure 1B is a schematic representation of the detection of an immunological reaction (ELISA) with a flag molecule: fluorescent DNA is used here as a reaction marker. Subfigure 1C is a schematic representation of genetic cartography or physical mapping using the extension of DNA and the use of a marker DNA.
Subfigure 2A and Subfigure 2B are fluorescence video micrographs showing, respectively, λ-DNA cohesive end-labeled with DIG on a surface covered with anti-DIG and stretched by the meniscus, and as a control, non end-labeled λ-DNA on an anti-DIG surface.
Figure 3 is a schematic representation of the combing of DNA molecules by passage of a moving meniscus.
Figure 4 represents a histogram of the measured lengths of an ensemble of λ-DNA molecules and its multimers, after ligation.
Figure 5 shows a histogram representing the measured lengths of an ensemble of λ-DNA molecules, obtained by mechanically translating one cover glass surface S relative to the other S'.

### DESCRIPTION OF THE PREFERRED EMBODIMENTS

This invention arose by the desire of the inventor to improve on prior art surfaces that are afflicted by highly inconsistent results produced by extraneous interactions between their chemical structure and substances present in a medium placed in contact with the surfaces. The present invention relies on the preparation and use of highly specific surfaces by, for example, eliminating parasitic binding and non-specific adsorption of macromolecule (s) to the surface, which may be utilized to immobilize macromolecule (s) for their counting, length measurement, study of their characteristics and their relationship or of fragments thereof to vicinal segments, and utilization in the preparation of sequence ready maps, genetic analysis of individual molecules, diagnostic and prognostic tests. The resulting surfaces result in improved surfaces with very limited background noise.

More particularly, the present invention concerns a highly specific surface suitable for binding macromolecules, for example, for use in biological reactions. The surface of the invention preferably comprises at least one layer of an organic compound presenting an exposed or reactive group having affinity for one portion or site, particularly an extremity or end section, of a macromolecule, e.g., a biologically active molecule. The reactive group of the surface may optionally be chemically modified to bind any reactive group or moiety on the macromolecule. The remaining elements of the layer present very few possibilities for binding of any type or have substantially no affinity for the macromolecules under standard reaction conditions of the macromolecule (s) with the exposed or reactive group (s).

The following terms are being defined as they will be interpreted in the context of the present invention throughout this patent. "Affinity" refers to either chemical reactivity or absorption of a certain type, for any molecules or macromolecules bound to the exposed group or moiety of the surface, whether modified or not. The terms "fixation", "anchoring", and "binding" have substantially similar meaning, and are to be understood as designating the attachment of a molecule, macromolecule, portion thereof, or group or moiety to another group or moiety, molecule or macromolecule or fragment thereof, surface, support or substrate. The terms "combing, aligning, stretching, orienting and straightening" are in many instances used interchangeably, and designate the extension of a macromolecule or polymer, from a random or given conformation in solution to an aligned, linear and spatially oriented conformation. "Support or substrate" designates either a solid support or substrate or one constituted by a non-solid element such as a liquid or gaseous particle presenting, or covered by, a compact layer. A "compact" surface is one that substantially prevents the macromolecule to access any interior layer (s) and/or the support, in order to minimize non-specific interactions. Of course, this inaccessibility should take into consideration the conditions in which binding of the macromolecule occurs. Preferred among the surfaces of the invention are those having exposed group (s), such as unsaturated bonds, e.g., -CH=CH₂ double bond (s). These unsaturated bonds may be converted to other moieties, such as -CHO, -COOH, -NH₂, and -OH, or they may be used as such. Other reactive groups suitable for use herein are those found, for example, in branched or dendritic polymers, hydrocarbons, and fluorocarbons. Those will be presented in more detail below. The incorporation into the surface layer of other reactive groups or moieties, such as those comprising oxygen and chlorine, in addition to the reactive group or moiety (ies), must generally be avoided as they may introduce undesirable non-specific interactions and parasitic absorption. The surfaces of the present invention may be obtained through different processes. Examples are surfaces made of a layer of a carbonated polymer, preferably branched, at least about 10 nanometers (nm) thick, presenting a class of reactive groups such as those defined hereinafter, the rest of the layer comprising substantially all hydrocarbonated or fluorocarbonated groups, surfaces obtained by deposition or anchoring of one or more molecular layers, obtained by forming successive layers bound to one another by non-covalent bonds, e.g., Langmuir-Blodgett (LB) films or by molecular self-assembly (SAMs). The first type of surface may be obtained by polymerization of at least one monomer generating on the surface of the polymer the desired exposed group, by partial depolymerization of the surface of a polymer to generate the exposed group or moiety, or by deposition of the polymer. In this process, one of the monomers is preferably a C=C vinyl group, the formed polymer being, for example, a polyolefin or a polyenic derivative. Examples are surfaces of the synthetic rubber type, such as polybutadiene, polyisoprene or natural rubber. The highly specific surface of the second type, utilized, e.g., for biological reactions, is generally constructed by providing on an elongated structure or substrate an essentially monomolecular and compact layer of an organic compound having at least one fixation or anchoring group or moiety having affinity for the surface or support or substrate, and an exposed or reactive group or moiety having substantially no or little affinity for the support and the fixation group under conditions effective for fixation of the molecule to the substrate. The exposed or reactive moiety by itself, or after chemical modification following fixation, provides affinity or binds the macromolecule. Preferred layers are compact layers obtained with organic monomers or compounds, which are different than the exposed or reactive group (s) and susceptible to lateral reaction with each other to create transverse bonds or cross-linking. Such compact layers make the support substantially inaccessible to macromolecules or molecules in a medium deposited on the surface and, therefore, substantially eliminate parasitic reactions. The organic compounds preferably have a fixation or anchoring group or moiety at one extremity or end, and an exposed or reactive group or moiety at another position of the molecule, e.g., the other extremity or end. Other foreseeable embodiments are also contemplated herein where the fixation group is located, for instance, in the middle of the organic compound and has at each exposed groups at each end. In order to avoid parasitic reactions with the organic compounds other than with their exposed or reactive group or moiety, it is preferred to use organic compounds with relatively inert exposed and the fixation groups. For example saturated chains such as parafins may be utilized.

The surface (s) of the invention may be characterized according to (a) the support or substrate, (b) the molecule provided on the support, which has exposed or reactive groups and fixation or anchoring groups, and (c) the interaction between the support and the fixation group molecule. In one embodiment, the binding is non-covalent, particularly of the hydrophilic-hydrophilic or hydrophobic-hydrophobic type, as is the case in the Langmuir-Blodgett (LB) films (K. B. Blodgett, J. Am. Chem. Soc. 57, 1007 (1935)). In these types of surface (s), the exposed or binding group is either hydrophilic or hydrophobic, particularly an alkyloxyl, alkyl, or haloalkyl group, such as CH₃, CF₃, CHF₃, CH₂F, and the other group (the fixation or anchoring group) is hydrophilic. The binding may also be covalent, in which case the binding group will generally react chemically with the support. Particularly preferred binding groups include silanes, chlorosilanes, silanols, ethoxysilanes, methoxysilanes, silazanes, phosphates, hydroxyls, hydrazides, hydrazines, amines, amides, diazoniones, pyridines, sulfates, sulfonics, carboxylates, boronics, halogens, halide acids, aldehydes, thiols. More particularly, it is preferred to use binding groups susceptible to react in a transverse manner with an equivalent neighboring group to provide transverse binding, e.g., silane, and more preferably dichlorosilane, trichlorosilane, dimethoxysilane, trimethoxysilane, diethoxysilane, and triethoxysilane. The transverse binding or cross-linking may also be attained at various points across the thickness of the layer by polymerizing the monolayer with reactive groups, eventually present on the chain between the binding site and the exposed group. Thus, the bi-functional and tri-functional fixation group molecules allow a uni-dimensional or bi-dimensional polymerization of the layer. The type of binding groups utilized will, as is known in the art, also depend on the nature of the support. Groups of the silane type are well adapted for covalent binding onto glass and silica. The exposed groups, regardless of the surface, are preferably chosen from among vinyl, or aromatic radicals, tertiary or secondary amines, esters, nitriles, aldehydes or halogens. Particularly preferred are vinyl groups. Vinyl groups may be chemically modified after binding to yield, for example, methoxylic or carboxylic groups, or derivatives thereof, such as alcohols, aldehydes, ketones, acids, primary, and secondary or tertiary amines. The chains binding the exposed group to the fixation group are preferably chains bearing at least 1 carbon atom, preferably more than about 6, and generally between about 3 and about 30 carbon atoms. Thus, even if the substrate is not completely covered, it is mostly inert groups, not the substrate that are exposed. Both, in this case and when there is lateral coupling or cross-linking within the layer, whether ionic or covalent, highly organized layers are obtained by self-assembly, even if the initial surface only has a reduced number of active anchoring sites when compared to the number of molecules obtained in a compact layer.

The support or substrate may be made of any and all types of solid matter, but preferred are glass, silica, other polymers (natural and synthetic) and gold, with or without pre-treatment of the surface. When glass or silica are utilized, the use of known techniques of surface functionalization with silane derivatives is preferred, such as for example, the one involving the reaction: Si-OH + Cl₃-Si-R-CH=CH₂ gives Si-O-Si-R-CH=CH₂, wherein R is, for example, (CH₂)₄. The reaction may be run with pure solvents, and leads to a number of molecules having a C=C end exposed outside the surface. The term support or substrate is intended to include not only unique solid supports, but also particles such as silica powder and polymeric beads, as well as other substrate forms, such as fibers or structured supports, which may be rendered magnetic, fluorescent or colored, as known from various measurement techniques. The support is preferably chosen to be basically non-fluorescent when the detection is done using fluorescence. The surfaces obtained by processes (A) and (B) described above exhibit an important specificity, which is obtained because of (I) the presence of specific reactive sites obtained either from exposed groups or from the fixed molecule, (ii) a very weak rate of non-specific interactions caused by the absence of parasitic bindings (covalent binding, weak binding of the absorption type, or hydrophobic binding) (Commercially available surfaces, such as Nunc^{R} and Costar^{R}, present numerous non-specific interaction sites, macromolecules, such as DNA, may be undesirably anchored at multiple sites, whereas on the surfaces according to the present invention, the macromolecules are anchored solely by their extremities), (iii) a very weak intrinsic fluorescent rate when required and a background noise of fluorescence weaker than the fluorescence signal of the single molecule to detect, (iv) the possibility to detect isolated molecules with a signal-to-noise ratio (SNR) independent of the number of molecules, using various techniques which are described below. These are mostly based on the identification of the presence of a macroscopic marker having a weak non-specific interaction with the surface.

The thus obtained surfaces are preferably further bound to or coated with biologically active molecule (s), such proteins, nucleic acids, lipids or polysaccharides and derivatives thereof. Examples of proteins are antigens, antibodies, ligands, cell receptors, enzymes, transmitters, products such as avidin or streptavidin, and derivatives thereof. Examples of nucleic acids are RNAs and DNAs, as well as the α, β, and thiol derivatives of these compounds, and mixed compounds such as the PNA's. It is also possible to bind mixed compounds such as glycopeptides and liposaccharides, or other substances, such as viruses and cells, or chemical compounds such as biotin. The biological molecules may be covalently or non-covalently bound to the surface, such as by absorption, hydrogen bonding, hydrophobic interactions and/or ionic interactions. It is possible to cross-link the bound molecules, e.g., biological grafted molecules, by known techniques to reinforce their cohesion ("Chemistry of Protein Conjugation and Cross-linking," S. C. Wong, CRC Press, 1991). As previously mentioned, it is possible to have an exposed or reactive group on the surface, which reacts directly with the molecule (s). The exposed group, however, may also be treated after being bound to the substrate, and converted to hydroxyl, amine, alcohol, thiol, aldehyde, ketone or -COOH prior to the molecule being affixed thereto. Once the surface has been provided with such exposed group (s), the macromolecules, e.g., polymers in general, proteins, RNA, and/or DNA, may be bound thereto by techniques known in the art. These reactions are generally conducted on surfaces which are already in use for biological analyses, in particular Costar^{R} or Nunc^{R} surfaces, or microbeads such as Estapor^{R}, Bangs^{R}, and Dynal^{R} for instance, on which macromolecules, particularly biologically useful molecules such as DNA, RNA, PNA, proteins, and antibodies, are anchored.

The surfaces of the invention are reactive with non-ionized acids such as acids containing the COOH and PO₃H₂ moieties, whereas they are substantially nonreactive with their respective anionic species. This particular property allows the anchoring of nucleic acids or proteins within a defined range of pH, often where the reaction speed is easily controlled by the pH, as well. Hence, for example, the anchoring reaction may be undertaken within seconds (if not limited by diffusion), for a macromolecule, such as DNA, present in a medium A at pH 5.5. In some instances, however, it may be preferable to allow the reaction to proceed for longer periods of time (2 hrs). In a pH 8.0 medium, however, the same reaction results in very weak anchoring. This pH dependent anchoring effect is a considerable improvement over the prior art, especially when compared to other surfaces which require the pretreatment or functionalization of the macromolecule for binding to the surface. In the case of DNA, for example, by addition of biotin, DIG, NHS, and the like, or other more specific reactants, such as carbodiimide, dimethyl pimelidate, and the like, which can produce a peptide or phosphorimide bond between -NH₂ and-COOH or -POOH.

The surfaces comprising C=C and other molecule and macromolecule binding moieties may be used for the direct anchoring of molecules, e.g., proteins, such as protein A, anti-DIG, antibodies, streptavidin, etc. The activity of the molecule may be preserved while the surface, which originally contained C=C groups, is thus modified to selectively bind any macromolecule of interest. It is therefore possible to switch from a surface of relatively broad reactivity to one of highly specific reactivity, which selectively binds macromolecules with affinity for specific sites on a protein. By grafting onto the surface a specific antibody, such as for example anti-DIG, a surface which originally had limited reactivity towards the antigen (DIG), now has a selective affinity for it, since the initial chemical groups have been rendered inactive by the grafted antibodies. It is also possible to graft onto the original reactive surfaces, either chemically or biochemically, other biological molecules, such as viruses, or other components such as membranes, membrane receptors, polysaccharides, and PNA's. It is also possible to bind the product of a biological reaction (such as PCR) on the prepared surfaces, and thereby, determine whether or not the reaction occurred or the extent to which it occurred, such as by determining the number of product molecules produced.

The present invention, thus, relates to highly specific surfaces for applications to kits and assays, such as qualitative and quantitative assays for macromolecules, biological reactions, and the like. The surface of the invention comprises an essentially compact layer of an organic compound having an elongated structure, optionally affixed onto a support, the layer having at least a fixation or anchoring group (s) for binding to a support, and an exposed or reactive group or moiety having low or substantially no affinity for the support and the binding or anchoring group under conditions effective for binding the latter two to one another. The reactive group being capable, particularly after subsequent chemical modification, of binding molecules, such as biological molecules or molecules selectively binding macromolecules.

The invention also concerns applications of the surfaces of the invention to the detection of macromolecules isolated with specific molecules or reactants, more particularly in detection methods having a SNR which is independent of the number of molecules detected.

The invention also relates to a reproducible method of orienting, aligning, straightening or stretching a macromolecule on a surface, comprising placing a macromolecule in contact with a surface S and a medium A, such that one site of the macromolecule is bound to S and a portion thereof is placed in A; and mechanically translating the contents of A relative to S under conditions effective to orient, align, straighten or stretch the macromolecule. The method of the invention is simple and novel, and in one of its embodiments it moves the macromolecule (s) and a triple interface line S/A/B, resulting from the contact of medium or solvent A with a surface S and medium B, along the surface S in the form of a meniscus. Although the macromolecule need not be completely soluble in the medium A, in one specific embodiment, the macromolecule is soluble in the medium.

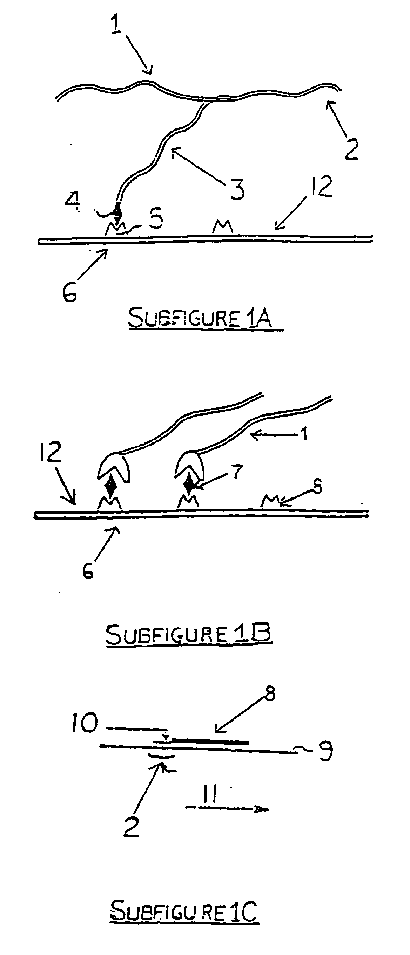

In accordance with the present invention, the inventor has observed that the simple passage of the meniscus on macromolecules, which are anchored, e.g., at one end, onto a substrate, permits an alignment which is substantially uniform and in a direction substantially perpendicular to the direction of movement of the meniscus, which leaves the molecules "combed" dried and absorbed on the surface behind the meniscus. When a fluorescence video micrograph was taken, it showed the extension of phage λ-DNA by the movement of the meniscus (Picture not shown). On the left, DNA molecules were seen still in solution before being combed or stretched by the meniscus, and on the right, DNA molecules were observed after the passage of the meniscus (See, Figure 1, Bensimon et al., Science 265: 2096 (1994). More particularly, the stretching of the free end of the macromolecule (the portion placed in the medium A) takes place as the triple interface line S/A/B moves along the surface S at the interface of the media A and B, which may be a gas, such as air and the like, or another discontinuous medium, including a solvent. In one particular embodiment of the invention, the meniscus comprises an air/water interface wherein, for example, the medium. A comprises an aqueous solution and the medium B comprises air. Other combinations are, of course, also contemplated. It is possible, furthermore, to extend the use of the air/water meniscus to other systems such as water/oil, water/surfactant/air, water/amphiphile/oil, and many others. The displacement of the meniscus may be accomplished using any means for the relative displacement of media A and/or B with respect to the surface S. This encompasses all forms of input energy, including but not limited to, thermal, electrical, mechanical, chemical, and other forms which will displace the A/B interface along the surface S. In particular, the meniscus may be displaced utilizing mechanical means, particularly by sucking or blowing a gas or by pushing or sucking the media A and/or B. The displacement of the meniscus may also be attained by progressive evaporation of the medium A. When the displacement of the meniscus is undertaken by mechanical means, this may be attained by translation of either the A/B interface or the surface S.

In one particular embodiment of the invention, the medium A is placed between two surfaces S and S', which may be part of supports or substrates, and the meniscus may be displaced either by evaporation or by moving either surface with respect to the other, or both. The term support, as used herein, designates any substrate which has a cohesion that is sufficient or effective to resist the passage of the meniscus. In another embodiment of the invention, voltage or electric potential changes or gradients may also lead to the movement or control of a medium, such as a fluid. Thus, an electrical means is one of the most preferred embodiments for translating the interface, for example on a "biochip", or in a microanalytical laboratory chip, such as one formed of silicon, or one of the micro or nano variety. The stretching force has been observed to, in accordance with the invention, act locally in the immediate neighborhood of the meniscus. This force is generally independent of the length of the macromolecule, or the number of macromolecules anchored, and in a large range, of the speed of the meniscus. These characteristics are particularly effective and important, and result in the aligning of the macromolecules in a homogeneous, reproducible, and quantitative manner. The present invention allows the preparation of surfaces, and substrates, other constructs and devices presenting on their surfaces, oriented macromolecules. Such a surface, substrate, construct or device may for example find applications in electronics or optics, as biosensors or biochips utilized for measurements based on their electrical or photonic properties. A uniaxial orientation may be best achieved by a uniaxial mechanism, e.g., translation, whereas evaporation typically leads to a broader or wider and more random distribution of oriented molecules.

It is possible to control the quality or degree of the stretching or combing of the macromolecules by using, for example, more than one different parameter, including (i) the interactions between the macromolecules and the surface, and (ii) the local action of the meniscus on the macromolecules. Thus, the stretching force is generally increased when the interaction energy between the macromolecule and the surface is large. In the case of a hydrophilic or wetting surface, e.g., one having anti-DIG bound thereto, this interaction energy is generally small, and thus the stretching force on the DNA molecule is not large. In one embodiment of the invention, the strength of the stretching force is voluntarily limited to avoid the breaking of the macromolecule, or the breaking of its bonds with the surface. Changes in the hydrophobicity/hydrophilicity of the surface, thus, generally lead to defined changes in the degree of combing or stretching of a macromolecule. In one embodiment of the invention, modifications are made on the surface moieties and/or the molecules bound thereto, which provide pre-determined changes in the degree of stretching for different macromolecules. It is also possible to attain a similar effect, in accordance with the present invention, by adding surfactants, amphiphilic molecules, polymers and co-polymers, e.g., block co-polymers, and/or other asymmetric substances into the media A and/or B to modify the properties of the interface which, in turn, will also modify the degree of stretching of a macromolecule. Thus, the degree of stretching of a macromolecule may be varied by either the addition of amphiphilic substances, e.g., surfactants, to the media, or by an appropriate surface treatment. Thus, stretching DNA in water/air (media A/B) on a hydrophobic (non-wetting) surface, such as silanated glass, results in about 50% stretching, whereas a similar test conducted on a hydrophilic (wetting) surface (covered with Protein A and anti-DIG), resulted in only 10% stretching (See, Bensimon et al., Phys. Rev. Lett. 74: 4754 (1995). An excessive surface/molecule attraction (for example an elevated level of adsorption) may be detrimental to the alignment of the molecules by the meniscus in accordance with the present invention because the molecules may remain absorbed to the surface in a state that is not necessarily an elongated or combed state. Preferably, the surface presents a weak absorption rate for the macromolecule, such that only the anchored molecules will be combed or aligned, while the other molecules are being carried away by the meniscus. Once the molecules are aligned, they adhere strongly to the surface. In the case of DNA, for example, they were observed extended several months after their alignment. The macromolecules may be anchored to the surface using the techniques described previously. Their binding to the surface may be either covalent or non-covalent depending on whether it results from a chemical or physico-chemical interaction (s). It is also possible to use commercially available surfaces, although these generally require pretreatment or functionalization of the macromolecules, e.g., DNA, prior to be anchored. The process of the present invention not only allows to observe and/or to quantify biological molecules, but also to separate biological molecules, using for example coupling techniques such as antigen-antibody or DNA-RNA coupling. In particular, the present invention relates to a process for the detection of a macromolecule comprising a DNA sequence or a protein in a sample, comprising placing a macromolecule in contact with a surface S and a medium A, such that one site of the macromolecule is bound to S and a portion thereof is placed in A; and mechanically translating the contents of A relative to S under conditions effective to orient, align, straighten or stretch the macromolecule. In the case of nucleotides, for example, it may be conducted by taking a sample corresponding to medium A comprising the macromolecule, preferably in solution, and placing it in contact with the surface, under conditions that favor the formation of a DNA-DNA hybrid, the formation of a DNA-RNA hybrid, or the formation of a protein-protein reaction product; the hybrid or reaction product is then suitably stained, stretched or combed, e.g., by the displacement of a meniscus, created by the contact of the medium with the surface, to give a particular orientation to the macromolecules, and the thus oriented macromolecules are then measured, observed, or detected.

The passage of the meniscus, which linearly stretches the molecules in the form of small sticks, renders them more easily detectable if they are stained (fluorescently for example). As the labeled macromolecules present a correlatable signal which has a uniform orientation by their stretching, they are effectively distinctive from the background noise. It is, therefore, easy to discard the presence of dust and non-homogenous elements, which do not present a particular spatial correlation. The ability of aligning macromolecules is of extreme importance because agglomerated molecules placed in a medium generally fluctuate from a thermal point of view, which leads to significant variations in the resulting fluorescence signal, and limits their observation and quantitative measurement. The present invention allows the observation of isolated molecules with an improved SNR. Stretched macromolecules may be revealed by different enzymatic methods or other methods, such as fluorescence or the use of "warm" or "cold" probes. Their detection may be attained by measuring of a global signal, e.g., total fluorescence, or by individual observation of the macromolecules by fluorescence optical microscopy, electron microscopy or methods using scanning probes, such as Scanning tunneling Microscopy (STM), Atomic Force Microscopy (AFM) or Nearfield Scanning Optical Microscopy (NSOM).

In a general way, the present invention allows the observation, separation and/or measurement of a quantity of a biologically active macromolecule in a sample by a process, using a surface onto which a molecule capable of recognizing the macromolecule of the sample is bound. The observation, separation or quantity measurement may be conducted using fluorescent or non-fluorescent reactants to detect the presence of either the bound molecule or the macromolecule. It is important to distinguish between the fluorescent and the non-fluorescent reactants. The fluorescent reactants contain fluorescent molecules, often chosen to be of a length about 0.1 µm or more, and specifically reacting either directly or indirectly with the pretreated surfaces. As a non-limiting example, a double stranded DNA molecule stained with fluorescent probes such as ethidium bromide, YOYO-1 or fluorescent nucleotides, may be directly anchored onto a surface exposing C=C bonds, or by modification of the DNA molecule (end labeled with DIG, biotin, etc.) on a surface having complementary proteins bound thereto (anti-DIG, streptavidin, etc.). The non-fluorescent reactants comprise, particularly, beads anchored through a molecule specifically bound either directly or indirectly to the pretreated surface. Because of the treatment of the surfaces, those beads present a weak non-specific interaction with the surface. Although non-limiting, examples are Dynal^{R} beads covered with streptavidin and anchored to a biotinylated DNA molecule by one extremity, and to a surface having sites capable of reacting with the other extremity of the DNA molecule. Depending upon whether the sought after macromolecule is detected directly by fluorescence, or indirectly by using the above mentioned reactants, either direct detection or indirect "flag" detection may be utilized.

In order to limit the problems associated with slow reaction times, it is possible to reduce the diffusion time of the reactants towards the surface by using small reaction volumes. For example, this may be done by conducting the reaction in a volume of a few microliters, which determines the spacing between two surfaces of fixed area, one of which is treated to present reactive sites according to the present invention and the other either is inert or treated. The detection of the number of the specific reactions which took place may be effected on a small number of molecules, typically 1 to 100, by a low noise macroscopic physical test requiring neither electronic microscopes, nor radioactivity or polymerase chain reaction (PCR) amplification. Depending on which type of reactant is used, two different embodiments of the invention (embodiment X and embodiment Y) may be used for their low noise macroscopic detection of the small number of anchoring reactions. In embodiment X, a test of the number of specific reactions that took place is directly obtained by a fluorescence technique which allows for certain embodiments of the invention to identify individually the number of sites which have reacted. In this case, the surface is chosen particularly to present a very weak rate of fluorescence. Particularly, the surface, or support must present a weak fluorescence. Once the anchoring of the fluorescent reactant has taken place, the detection and the counting of the number of anchoring reactions may be done using a fluorescence optical microscope having a wide numerical aperture objective, allowing to identify either directly with the naked eye or after having acquired the signal through an intensified CCD, the number of anchored fluorescent macromolecules, or alternatively with a photometry system. Particularly, it is possible and preferred to scan the observation field of view, in order to explore the total surface of a sample, which is often greater than a single observation field of view. In embodiment Y of the present invention, a macroscopic reactant of the bead type is detected. The novelty and unobviousness of this process is principally related to the use of beads having a specific reactivity, the use of beads having a size of between 0.1 and 200 µm which can be detected by a macroscopic technique, and the absence of a non-specific reaction between the beads and the surface, which is due to the use of the product in accordance with the present invention. The number of these macroscopic beads each characterizing an anchoring may then be determined by a macroscopic physical method, e.g., diffusion of light on the beads, optical microscopy and/or the fluorescence of the beads, among others.

The specificity of certain biological reactions can be limited. Thus in a context of hybridization, the hybrids may be imperfect while presenting a reduced number of matchings and therefore a lower quality of binding. The present invention also covers the possible use of a test step to detect the quality of the binding obtained. This test allows the dissociation of products linked in a non-specific way by absorption, by hydrophobic forces, by imperfect hydrogen bonding and by imperfect hybridization, among others. The present invention also expands the application of a process of detection or measurement, such as the one described previously, to a process in which the reaction product between the molecule and the macromolecule of the sample is submitted to a physical constraint in order to destroy the weak bindings prior to the detection. This process offers the possibility to destroy the wrongly bound couples and the possibility of orienting the coupling products. This facilitates the measurements or the observations. In this context, it is possible to apply to the surfaces, after binding of the complementary elements, a constraint e.g., the single or combined use of either centrifugation, a magnetic field, agitation, the passage of a meniscus, electrophoresis and/or variation of temperature to dissociate undesirable bonding. It is important to note that due to the use of the surfaces of the invention, it is possible to orient the molecules after their binding by passage of an air/water meniscus, particularly on DNA.

Thus, by applying the method of the invention the translation of a macromolecule bound to a surface , e.g., DNA, in air/water media results in a regular extension of the anchored molecules. These remarkable and unexpected observations show that it is entirely possible to count the number of macromolecules, such as DNA, anchored to a surface. On one hand, the surfaces used may be only slightly fluorescent while providing a relatively low signal to noise ratio (SNR). On the other hand, because the presence of an extremely correlatable object is sought after (in the form of sticks), it is very easy to increase the SNR. In other words, it is possible to ignore dust particles and non-homogeneous portions which do not present a particular spatial correlation. It is notable that the SNR attained by the present invention is independent of the number of anchoring reactions. The SNR in the detection of 1 molecule is the same as it is for 10,000 molecules. Furthermore, the stretching or combing method of the invention allows to discriminate easily between molecules having different lengths. It is additionally possible to use steps, e.g., the following steps, to further increase the signal to noise ratio: (I) the molecule being fixed, it is possible to integrate its fluorescence signal, (ii) the observation with a reduced field microscope (typically 100 µm x 100 µm with an oil immersion objective of N. A. 1.25). For a sample of one centimeter squared it is possible to proceed by either scanning, or through the use of a lower power objective with a larger field of view, (iii) the sticks always being parallel, it is possible to use an optical spatial filtering method to further increase the SNR, (iv) other global fluorescence methods can be used, e.g., EP Application 103426, (v) the stretching of the molecules in straight lines is observed either in the context of a chemical grafting or in the context of immunological binding, (vi) once the surface is exposed to the air, the DNA molecules are stable and fluorescent for many weeks. It is possible to use this property to differ in time the anchoring step from the analysis of the anchored molecules, if this detection is made by fluorescence microscopy, (vii) a double fluorescence technique can also be used to improve the SNR or to detect a double functionality.

This invention also provides an instrument, apparatus, reader, or dedicated device to make observations, determinations or measurements of oriented macromolecules bound to a surface. The instrument generally includes, but is not limited to, a light source (s), sample (s) holder, optical arrangement and detector (s).

The alignment and detection techniques described in accordance with the present invention may be used for numerous applications including, but not limited to, the identification of one or more sequencing elements of DNA or RNA which can be advantageously used for the diagnosis of pathogens or for genetic cartography and physical mapping, the measurement of the distribution of the length of extended DNA fragments can also be used for genetic cartography and physical mapping, the improvement of the sensitivity of the ELISA techniques with the possibility of detecting a small number of immunological reactions. The identification of DNA/RNA sequences may be done either (a) by reaction in the volume of the solution of DNA/RNA molecules with complementary probes (for example by hybridization) or (b) by specific proteins of the sought after segment. Thus two possible operating modes are possible.

This invention also relates to a kit for the detection of the presence of a molecule of interest in a sample or the position of a portion of a molecule of interest within a larger molecule. The kit generally comprises, in addition to instructions for its use, a surface binding macromolecule of interest by at least one anchoring point, reagents necessary to either identify, reveal or anchor the molecule of interest. Examples of reagents are, either alone or in combination, buffers, probe or marker molecules (DNA, RNA, proteins, ...), fluorescent stains, the specific binding molecules, e.g., complements to the macromolecules, such as ligand-receptor, biotin-streptavidin, and optional means for conducting a non-evaporative movement of the meniscus (for example mechanical shearing means such as a second surface to be applied on the surface described in (i) as described previously. The shearing means are optional because it is possible to let the sample evaporate (in time).

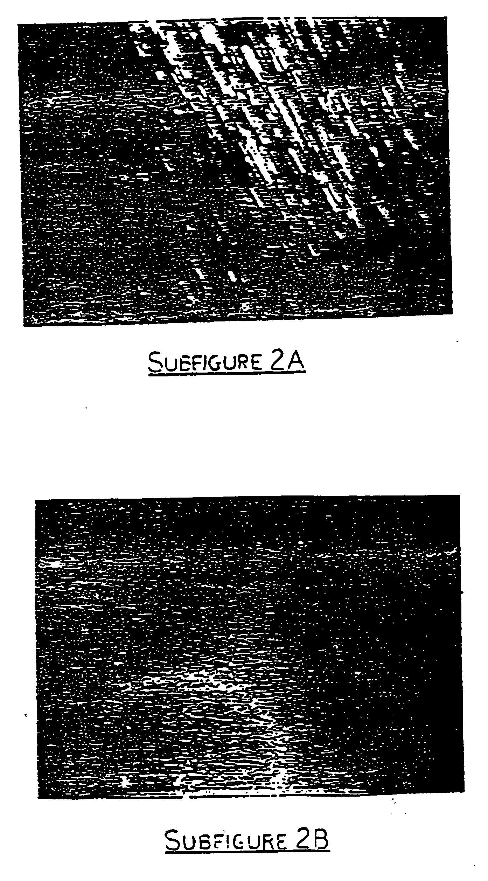

Given that only the molecules anchored to the surface are stretched by the meniscus, and the medium and the other molecules contained therein are carried away by the meniscus, the method of the invention may be used to detect the presence of a selective or specific anchoring between a molecule and the surface, and hence of a biological reaction, such as an ELISA reaction. Thus it is possible to use fluorescent DNA or other polymers as a reaction marker (for example by coupling the DNA to an element recognizing an immunological reaction of the ELISA type) and to estimate the number of reactions by counting the number of molecules stretched on the surface, (the detection threshold is less than 1000 reactions). It is also possible to use the present invention for the detection of the presence of a pathogen, for instance by the hybridization of DNA to a complementary oligonucleotide, capable of being anchored specifically to a surface, and then counting the DNA molecules stretched on the surface. As for molecular cartography or physical mapping, the stretching of a molecule can also be used to detect a given pattern along this molecule, for example, as it is done in genetic cartography. For instance, a marker DNA having approximately 3,000 base pairs and a single stranded extremity complementary to the sought after gene along the molecule, may be dyed with a fluorescent marker A. This marker DNA may then be hybridized with a single stranded DNA from the molecule from which a cartography is required, and the complex obtained is then dyed with a second fluorescent substance B (after a random priming reaction to transform the hybrid into double stranded DNA). The complex may then be anchored by one of its extremities and stretched by the action of the meniscus. The distance between the extremity of the molecule and the position of the marker, which can be observed in double fluorescence microscopy (two colors A and B), renders it possible to establish the position of the sought after gene with a precision of the order of 1000 bp. Another possibility is to use the techniques of FISH and DIRVISH (I. Parra and B. Windle, Nature Genetics 5: 17 (1993)) to perform the physical mapping or cartography on already stretched molecules. However, since the force the meniscus exerts on a DNA molecule or polymer is sufficient to stretch it beyond its natural or crystallographic length, it is necessary to calibrate the distances measured on a surface (see, for example, Fig. 2 of A. Bensimon et al, Science 265: 2096 (1994). For example, if the natural length of a molecule is lₒ (3.3 Angstroms/bp), 16.1 µm in the case of λ-DNA, then if the peak of the histogram of measured lengths of the population of λ-DNA was measured to be lₚ = 24 µm, the relative extension, due to the passage of the meniscus is lₚ/lₒ = 24/16.1 = 1.49 or a 49 % relative extension. Thus all measurements of distance, typically made via fluorescence based microscopy, as mentioned in the above and below examples, must be corrected for this relative extension factor, before any true or accurate distance or position determinations can be made. It is often desirable to average measurements and integrate signals in order to more accurately quantify observations. Peaks of histograms often convey the mode of a distribution useful in measurements and quantification. This may specifically be accomplished through direct or indirect calibration. In the indirect mode, one of a batch of equivalent surfaces is calibrated for the relative extension factor and is then used and applied for all the surfaces of the batch. The danger with this approach is that it was observed, at least in the case of silanated surfaces, that there can be surface to surface fluctuations in the degree of extension, which would become a source of error in distance or position determinations. In the direct mode, a small quantity of a known stained standard length molecule (e.g., λ-DNA, a large fragment thereof, or marker) may be added to the aliquot to be tested on a surface. After combing, the relative extension of the population of standard length DNA is measured to determine the exact relative extension factor for this surface and conditions of meniscus passage, and this factor is used only for distance or position determinations on that surface. This increases the work load, but reduces the sources of error.

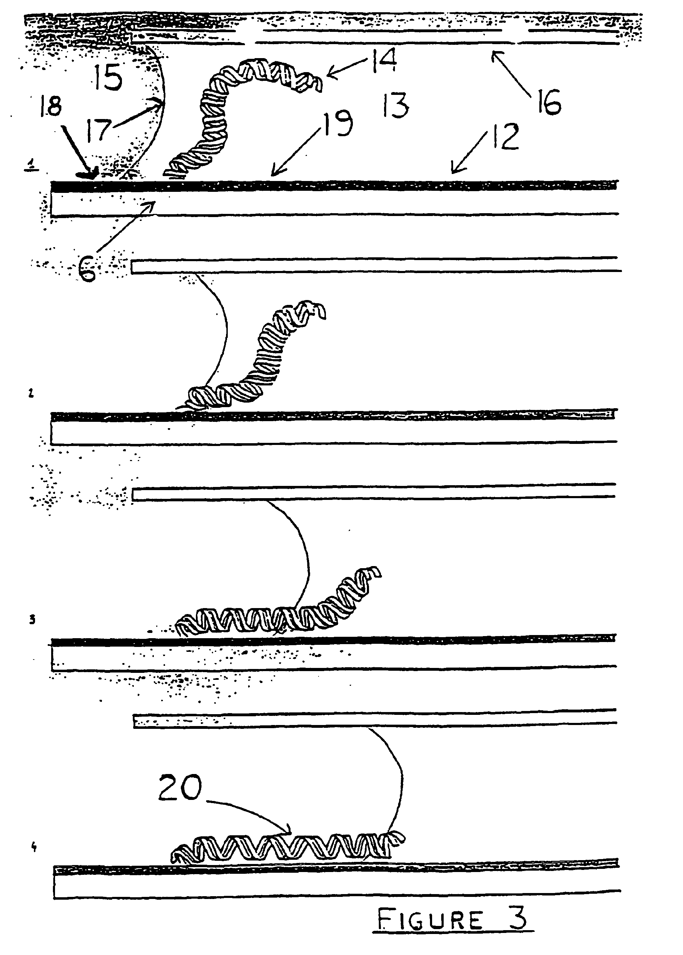

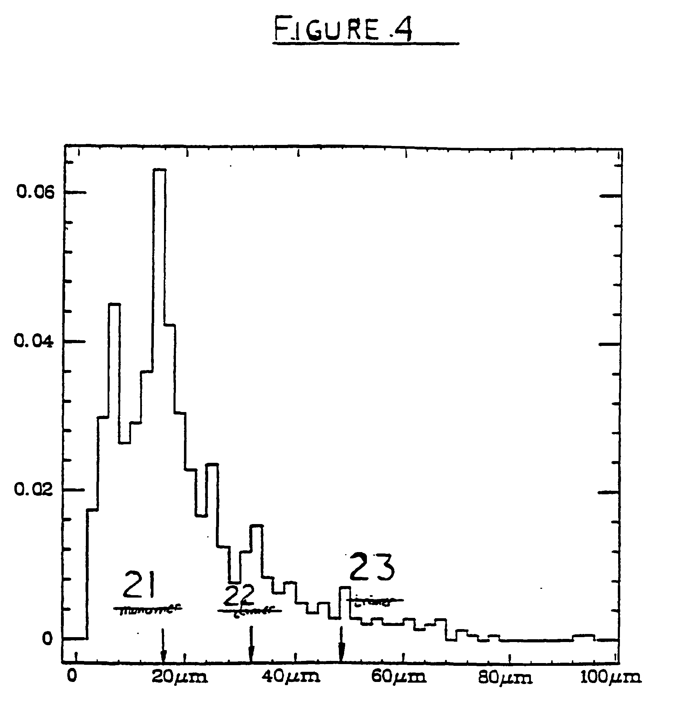

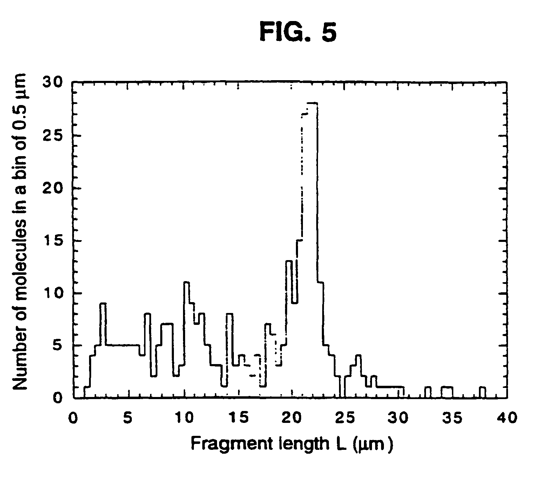

The present invention also relates to a surface, and to a substrate, construct, or device provided with at least one such surface, where the surface has bound thereto an aligned macromolecule obtained by the stretching process described above. In particular, it is possible to obtain surfaces having electrical or optical properties, such as may be used in "biochips", biosensors, or microanalytical laboratories in silicon-type devices of nano fabrication and lithography: The following description will be made with respect to the enclosed drawings in which Subfigure 1A is a schematic representation of the detection of a pathogen in a fluorescent DNA molecule by hybridization with an anchored molecule. This subfigure shows a fluorescent DNA 1, a complementary sequence 2, an anchored molecule 3, an antigen DIG 4, an antibody anti-DIG 5, a substrate 6, and a surface 12; Subfigure 1B is a schematic representation of the detection of an immunological reaction (ELISA) with a flag molecule: fluorescent DNA is here used as a reaction marker. This subfigure shows a fluorescent DNA 1, a substrate 6, a macromolecule to be detected 7, an antibody 8, and a surface 12; and Subfigure 1C is a schematic representation of genetic cartography or physical mapping using the extension of DNA and the use of a marker DNA. This subfigure shows a complementary sequence 2, a DNA marker (color A) 8, an anchored DNA (color B) 9, a position of overlap 10, and a direction of stretching 11. Subfigure 2A is a picture of a fluorescent video micrograph showing λ-DNA cohesive end-labeled with DIG on a surface covered with anti-DIG and stretched by the meniscus, and Subfigure 2B is also a picture of a fluorescent video micrograph showing a control of cohesive non end-labeled λ-DNA on a surface covered with anti-DIG. The very narrow specificity of the surfaces and the absence of non-specific absorption can be noted. Figure 3 is a schematic representation of the combing of DNA molecules by passage of a moving meniscus. This figure shows a surface 12, to which is attached a macromolecule (DNA) 14 placed in medium A 13, next to medium B 15, and opposite the surface is a second surface 16, and interfaces A/B 17, B/S 18, and A/S 19. The stretched molecule 20 is also shown attached to surface 12. The DNA solution is covered with a circular non-treated cover glass. Figure 4 represents a histogram of the measured lengths of an ensemble of λ-DNA molecules and its multimers, after ligation. The major peak of the distribution corresponds to the length of the monomers 21 (16 µm), whereas one can identify peaks which correspond to the dimers 22 (32 µm), and the trimers 23 (48 µm). The other peaks result from the fractionation and mechanical shearing of the fragile DNA molecules during their manipulation. This figure demonstrates the homogenous character of the stretching, independent of molecule length. Figure 5 shows a histogram representing the measured lengths of an ensemble of stretched λ-DNA molecules, obtained by mechanically translating one cover glass (surface S) relative to the other S'. A peak at 21 µm indicates stretching from the crystallographic length of 16 µm. This sample was identical to another sample containing the same DNA bound to a silanated surface, except that it was combed or stretched by evaporation of the medium A. Thus, this one may be directly compared to Figure 2 of Bensimon et al., Science 265: 2096 (1994). This provides a relative extension of over 30%, and a number of fragments of N =345.

When used in applications, such as for diagnosis, the probes (the anchors) may have a reactive group, e.g., DIG, biotin, etc., capable of being anchored in a specific manner to a surface in accordance with the present invention (having for example as an anchoring site, an anti-DIG antibody or a streptavidin). The detection of the anchoring reaction can be done directly by fluorescence of the DNA molecule stained by fluorescent molecules (ethidium bromide, YOYO, new fluorescent nucleotides) (See, Subfigure 1A). It may also be done indirectly by the detection of a flag molecule, e.g., a reactant in accordance with the present invention capable of being fixed on the DNA or RNA molecule (for example by hybridization or protein DNA interaction etc.), but having substantially no affinity for the anchoring sites of the probe.

In the cartography mode, the complementary probes are directly coupled to a fluorescent reaction in accordance with the present invention. It may be, for example, complementary single stranded DNA having modified fluorescent nucleotides, or a long single stranded DNA stained with fluorophore A and terminated by a complementary single stranded fragment of the sought after sequence. For different probes, fluorophores having different colors can be used. It is also advantageously possible to dye the DNA on which the probes hybridize with a fluorophore having another color. The DNA/probe hybrid is anchored by one of its extremities and stretched by one of the methods described previously. The distance between the anchoring points and the hybridization points or between the points of hybridization is determined by the detection of the fluorescence of the probe using the methods of the present invention described previously (See, Subfigure 1C). For example, without being limited thereto, a marker DNA, having about 3,000 base pairs and having at one end of its extremities a single stranded fragment complementary to the sought after gene, is stained with fluorophore A (e.g., YOYO-1). This DNA may be hybridized and ligated with the single stranded DNA for which a cartography is desired, and this later single stranded DNA is dyed with a second fluorophore B (POPO-1) (after a random priming reaction to transform the DNA into double stranded DNA). The molecule is then anchored by one of its extremities (for example by a DIG/anti-DIG reaction) and stretched by the action of a moving meniscus. The distance between the extremity of the molecule and the position of the marked gene, observable in double fluorescence microscopy (two colors A and B) allows one to establish the position of the sought after gene with the precision of the order of 1,000 base pairs (0.3 µm). The identification of DNA/RNA sequences can also be done by a reaction between the sought after sequence and reactive sites of a surface, in accordance with the present invention (for example, complementary oligonucleotides or the site of reaction of a specific protein of the sought after segment). The detection of the anchoring reaction may then be done directly or indirectly (with the help of a flag molecule) as described previously. It is to be understood that identification of sequences of DNA/RNA, in accordance with the present invention, can be used either for diagnostic purposes (for example for the detection of the presence or absence of a viral or chromosomal pathogen) or for genetic cartography and physical mapping purposes. It can be proceeded by an amplification step using various methods, particularly PCR. Genetic cartography and physical mapping can be accomplished by a measurement of the size of DNA fragments. This is possible because the binding to surfaces and molecule stretching techniques described above (in particular and advantageously the stretching by the meniscus) allow a measurement of the length of stretched molecules, even for a minute quantity or sample.

It is possible to proceed, but without being restricted to this process, in the following manner. A sample of DNA may be fragmented, e.g., with restriction enzymes, dyed with a fluorophore and anchored on a surface presenting reactive groups (e.g., surfaces with C=C groups). The molecules may then be stretched by the meniscus and the size of the stretched fragments is determined by fluorescence optical microscopy with a resolution, and limiting size, of the order of 1000 base pairs (0.3 µm). The results obtained may be shown as a histogram of the measured lengths of extended DNA molecules incubated at various fluorophore to base pair ratios (1:5, or 1:10 or 1:20 fluorophore: base pairs). The histogram shows that the stretching of the molecules beyond their crystallographic length is not due to the intercalation of the fluorophore, but is instead due to the stretching action of the meniscus (Histogram not shown). The surface of the invention, can be used in known processes allowing the identification and/or quantification of antigens or antibodies, particularly ELISA methods using enzymatic systems, or methods of the RIA type using radioactive markers. These techniques are well known and need not be described in detail herein. It is also possible to use the surface of the invention as a support of immunological reactions of an ELISA process having a reactant anchoring step in accordance with the present invention (flag) on one of the ELISA reactants (Subfigure 1B). The detection can be done globally by measuring the total fluorescence. It is also possible to count the number of reactions locally. This can be done using the detection methods of the present invention, in particular after the meniscus extension. This is possible because of the weak fluorescence rate and the small number of non-specific interactions of the product of the present invention. This also allows the detection of a very small number of reactions (eventually less than 100) with an excellent SNR. It is therefore possible, using minor modifications of sandwich ELISA methods (antibody-antigen modified, for example biotinylated), to graft on the surface a reactant in accordance with the present invention, for example fluorescent DNA anchored to streptavidin. All the variants of the ELISA technique can be applied with an improved sensitivity. Fluorescence measurement techniques are already used to determine the quality of ELISA reactions. However, the process of the present invention allows an improved detection sensitivity because it is sensitive to the fluorescence signal of a single molecule.

The present invention also provides a novel method and technology for blotting macromolecules, e.g., polynucleotides such as DNA, with ultralow fluorescence background, previously unknown. The present technology offers an alternative to conventional southern/northern blots, often utilized in molecular biology applications. At the present time, DNA fingerprinting may be cumbersome, particularly because of the need for amplifying the materials obtained. The fingerprinting of DNA samples must first overcome the barriers presented by the minute quantities of DNA, often limited by the size of forensic samples, and problems associated with low detection sensitivity. Presently available techniques are time consuming, typically requiring many weeks, and are complicated, requiring multiple exposure of radiolabeled blotted membranes. Conventional techniques rely on restriction cutting of DNA by an ensemble of enzyme markers (typically 5), each restriction cut is then loaded into a separate well on a standard high resolution gel; DNA fragments of various sizes are separated by gel electrophoresis in each lane resulting in characteristic patterns; and finally to determine each pattern, i.e., the "genetic fingerprint", each gel is then blotted onto an adsorptive and typically fluorescent membrane, transferring the DNA onto the membrane in its spatial location, and each marker is detected by subsequent radiolabelings, separate long-time exposure to a sensitive film (weeks), and washing of old radiolabel before the next label can be imaged onto the radiograph. The prior art process takes typically about 2 weeks per marker or about 10 weeks for a 5 marker fingerprint.

The present invention provides a novel and highly sensitive method for the detection and fingerprinting of minute quantities of DNA using fluorescence microscopy, in a relatively short period of time. The present method relies on the use of a solid non-fluorescing surface, optionally part of a substrate, (e.g., a glass cover slip with treated silane coating), onto which to blot macromolecules, (e.g., DNA or proteins). In one embodiment of the invention, a thin µm thick gel is placed between a treated and a non-treated surfaces, (e.g., silane surface with exposed C=C bonds), such as those that may be obtained from a cover glass. A sample containing macromolecules, e.g., polynucleotides and/or proteins, may be loaded onto the gel, and electrophoresis conducted at an alkaline pH, e.g., at pH 8.0, where there is little or substantially no binding of the macromolecule to the C=C bonds of the surface. The DNA/proteins are then electrophoretically separated, and the pH is lowered to promote binding of the reactive group in the macromolecule, e.g., to pH 5.5, under conditions effective to increase binding. e.g., in a 50 mM MES medium for DNA binding to C=C bonds. The samples may then be stained, and the gel temperature elevated in a manner sufficient to melt the gel medium, e.g., low melting point agarose, and the medium translated with respect to the surface to which the macromolecule has bound. This results, by passage of the meniscus, in a combed or stretched sample. The macromolecule state may then be observed by fluorescence microscopy, which results in a direct spatial fingerprint of the specific location in the gel of all DNA fragments at the time they bound to the surface. The present technology accomplishes the entire process in a short period of time, i.e., about 1 to 2 days, without needing to resort to PCR amplification. The present invention is an extremely sensitive detection method, and provides an optical spatial resolution of about 0.3 µm, and in some instances even better.

Another embodiment of the invention, the method may be conducted onto untreated surfaces, e.g., surfaces lacking C=C bonds, by layering a thin gel as is known in the art, and electrophoresing as described above, and then removing the second surface ("floating" the cover glass off), varying the pH of the gel to a pH value effective to promote binding, and blotting the gel directly, e.g., for polynucleotides at low pH, onto a different surface with exposed C=C groups to promote binding.

The fingerprinting method of this invention is fast, utilizes at least one non-fluorescent but reactive surface, e.g., a surface with exposed C=C or other reactive bonds, produces an optical microscopic fingerprint, is not macroscopic size technology, and results in advantageous high optical resolution utilizing a microscope at high magnification. This method relies on simple changes, such as changes in temperature, pH and other treatments of the gel after binding the macromolecules to the C=C or other reactive groups on the surface, or electrophoresing at one pH where substantially no binding occurs, and then fostering binding by dropping the pH. Clearly, this is a substantial improvement over the prior art, which is fast, simple to apply, and utilizes standard molecular biotechniques and combines them in a new and unobvious way.

This invention also extends to the use of minute quantities of a "marker" or "tag" as a means to authenticate samples, such as fluids. However, other samples may also be used by placing them in a medium A, or solubilizing them therein. Often, in commerce, there are problems in authenticating a given material, such as a fluid, to be of the make indicated on the label. Fraud often occurs in the merchandising of, for example, counterfeit perfumes, wines, champagnes, and other priced products. This aspect of the invention relies on the addition of minute quantities of known single and double stranded (ds) polynucleotides, such as DNA, RNA, and PNA, e.g., linear single or dsDNA, in amounts as low as about 10³ to 10⁶; preferably about 10⁴ to 10⁵, macromolecules to a volume of about 5 to 1,000 ml, for example in a bottle of perfume or wine, as an inert additive, which will be extremely difficult to detect and/or duplicate, in order to sell a fraudulent product as a substitute for the original. In one embodiment of this invention, a linear ds polynucleotide may be added in an amount of about 10³ to about 10⁶, and in some instances even in smaller quantities, having (i) a length of at least about several microns (greater or equal to about 10 kb), and (ii) one or more known or specific base sequence (s) which produce (s) unique restriction pattern (s) or fingerprint (s) when separated by an electric field, e.g., by electrophoresis on a gel of a fluid, or upon "optical mapping", as described by D. C. Schwartz (Meng, X, et al., Nature Genetics 9: 432 (1995). The polynucleotide may be added to a fluid sample, or if the sample is not in fluid form, to a medium A suitable for dissolving and/or suspending the sample to be analyzed, e.g., a perfume or wine. The addition of the "marker" may be undertaken during the manufacturing process or at the time of packaging for shipment at the original or authentic source. These molecules of an inert and unknown concentration and base sequence will serve as a "passive sealer" or "internal marker" or "tracer tag" to signify the authenticity of a product or liquid. It is well known that polynucleotides, e.g., DNA, may be broken down and eliminated by the gut upon their consumed via the gastrointestinal tract and, therefore, their addition does not appear to present any health risks to humans or other mammalians who might ingest them. Applications of minute quantities of polynucleotides, e.g., DNA, onto the skin or other parts of the mammalian, e.g., human, body are not known to present a health hazard either. In order to authenticate a product or to verify its origin, a minute sample, e.g., less than about 1 ml containing several hundred macromolecules, may be extracted for analysis. Each sample for testing, however, requires a much smaller volume, e.g., typically about 1 µl to 50 µl. This small sample volume may be placed in an Eppendorf tube, centrifuged, and concentrated, e.g., in a vacuum rotary centrifuge evaporator, and readied for analysis and detection. Various means of sensitive detection may be employed for this purpose, such as the ones described below. The ds polynucleotide may be resuspended in an appropriate inert medium, e.g., 50 mM MES pH 5.5 for a total volume of, e.g., about 20 µl, and placed onto a surface, e.g., a non-treated surface or a surface comprising C=C or other macromolecule binding groups, such as the one described above. The sample may then be directly subjected to macromolecular combing by binding one portion, preferably one end thereof, to the surface (e.g., a surface with C=C or other binding groups), or a surface S' placed on the sample, and the macromolecule translated, e.g., by electrophoresis, the surface S' removed and a surface S" comprising C=C or other reactive groups substituted therefor, and then combed or stretched in accordance with the teachings of this patent, e.g., by the passage of an air/water or other combination of media (interface), through evaporation or mechanical means, including shearing. The macromolecules may then be detected after staining, e.g., by fluorescence microscopy as previously disclosed. If desired, the sequence of the macromolecule may be designed to contain specific restriction sites, and made of an appropriate length, so that the macromolecules may additionally be subjected to restriction digestion by specific known cutter (s), e.g., restriction enzyme (s), and the resulting physical map may then be compared to a control for the similarly treated macromolecule, e.g., the expected or authentic fingerprint. This procedure may be done in reverse order, as well, wherein the ds polynucleotide may also be concentrated, then restriction digested in an Eppendorf tube, then placed in a medium, e.g., a thin gel at a pH where the macromolecule does not bind to the surface, e.g., pH 8.0, and electrophoresed to fractionate the fragments, the pH then changed to promote binding of the macromolecule, e.g., lowered to about pH 5.5, S" placed in contact with the medium A, e.g., blotting of the macromolecule onto a binding surface with a C=C or other binding groups, e.g., binding filter paper, and bound thereto, the temperature may then be elevated to melt the low melting point medium, e.g., agarose gel, and the macromolecule (s) or fragments thereof may be mechanically sheared to comb or stretch them. Alternatively, the dried and concentrated macromolecule, e.g., DNA, may be rehydrated, e.g., with a suitable buffered medium, and then the macromolecule (s) may be amplified by the polymerase chain reaction to produce normal or long range PCR (LR-PCR) products. These PCR products may then be detected, restriction mapped, or shotgun sequenced and compared to a control, in order to determine whether or not the sample's macromolecular pattern or sequence matches the known or expected authentic macromolecule's pattern or sequence. The following are some of the unobvious improvements provided by this invention over the prior art. This technology uses macromolecules of an extremely stable nature, e.g., DNA, as a means to authenticate the origin of different products, and very small amounts of the macromolecules are utilized and/or required, i.e., very low concentrations. In addition, the specific nature of the macromolecule is difficult to detect and duplicate, given that the exact sequence of the macromolecule must be known to do this, and the sequence of the macromolecule may be easily changed for each and every lot or shipment, in order to introduce more variability and thus difficulty to a potential counterfeiter. The choice of means for sensitive detection is novel and unobvious, as is the use of surfaces comprising C=C and other binding groups for macromolecular combing as a means of ultrasensitive detection, and the use of now conventional PCR and long range LR-PCR enzymes and protocols, and/or combinations thereof. The present technology allows manufacturers a simple means for deterrence and authentication or verification, and/or prevention of counterfeiting, of their products. This technology is simple to implement at the origin; simple to verify or read out using standard molecular biology techniques; and difficult to forge since exact duplication of a long dsDNA fragment is difficult to accomplish since the precise sequence of the macromolecule must be first determined and then cloned and assembled in sufficient quantity and with sufficient speed to proceed before that particular batch disappears from the market place.

Still another invention related to the use of the surfaces of the invention comprising C=C and other reactive groups or moieties described herein for the manufacture of structured substrates and devices. The reactive surfaces of the invention may be incorporated into structures such as "biochips" or "Microlabs", which comprise a "complete analytical microlaboratory" for a desired purpose on a chip. Such chips comprise a front end of sample preparation area, a fractionation or separation area, also typically a reaction chamber (s) where various reagents or products are added, and then typically a detection area for readout of the macromolecule (s) properties by, e.g., optical or electric means. The present surfaces are useful for the immobilization of macromolecules such as nucleic acids or proteins, among others, in a reaction chamber of such a "biochip", as well as as a means to effect separations by the action of a moving meniscus or a hydrodynamic flow or other constraint or force field. The surfaces of this invention may also be used for the detection or quantification of desired macromolecules, in the readout phase of the chip by, e.g., internal photonics (laser diode/photodiode), external (microscope), and the like. This invention finds one of its uses as a means to immobilize macromolecules to affect separation or identification of a given or desired macromolecule within a sample. It uses reactive surfaces within a structured device as well as a means to immobilize molecule (s) or macromolecule (s), to then expose the bound macromolecules to combing or stretching as described above to identify, separate and/or measure the quantity of a desired macromolecule.

Other advantages and characteristics can be appreciated by referring to the following examples.

### EXAMPLES

### Example 1: Materials and Methods

The λ-DNA and monoclonal antibodies (anti-DIG) were purchased from Boehringer-Mannheim. The trichlorosilanes were purchased from Roth-Sochiel. Fluorescent nucleic acid stains (YOYO-1) were purchased from Molecular Probes. ESCO pre-cleaned glass cover slips (22 mm²) were purchased from Erie Scientific. Magnetic beads were purchased from Dynal Corp. Microscopes used included (i) an inverted Nikon Diaphot^{R} with 60x/1.4 N. A. objective, equipped with a Xenon burner for epifluorescence, with an Omega Optical filter set and an ICCD image intensifier from Hamamatsu for video observation, or (ii) a custom built inverted microscope including an Olympus^{R} and focus mechanism, a Zeiss^{R} 100x/1.3 N. A. objective, an argon laser (488 nm line) to stimulate the fluorophore, an Omega optical filter set, and a DEP hybrid ICCD image intensifier.

### Example 2: Surface Treatment

Pre-cleaned glass cover slips were removed from the box using tweezers, and placed in custom built Teflon^{®} carriers, designed to support each cover glass vertically with a minimum spacing of at least 2 mm. These racks of roughly 20 cover slips were then loaded into a special reaction chamber designed for both cleaning and silanation, approximately 2 liters in volume, and flushed with pure O₂ for 10 minutes at a flow rate of 4 l/min. Organic cleaning of the surfaces occurred by illuminating an UV source within the chamber, creating ozone which reacts with the surface organics, for typically a minimum of 4-6 hours, with the best results from cleaning periods being much longer (12 to 72 hours). After purging the ozone (in a fume hood) for 5-10 minutes with dry O₂, the cleaned slides were then hydrated with a controlled humidity of moist O₂, generally from about 0.01% to about 30%, and sometimes even higher, typically 10%, RH atmosphere for 20 minutes at a reduced flow rate of 250 ml/min, in order to allow water molecules to bind to the surface. The water molecules are a necessary chemical species in the silanation reaction for the trichlorosilanes used herein. Then, 100-200 µl of a trichlorosilane Cl₃-Si-(CH₂)_{N}-CH=CH₂, was introduced into the chamber through a tiny port by a stainless steel syringe needle so as to not disturb the controlled internal reaction conditions. If desired, this may be attained by pumping on the chamber to vaporize silane, or heat it to increase the diffusion of the coating material. The surfaces were coated overnight (12 hours) for N=6 or rapidly (1 hour) for N=1. Upon removal, the surfaces often appeared slightly cloudy and were hydrophobic (that is non-wetting) yielding large contact angles of roughly 90 degrees for a drop of water. Surfaces were stored wrapped in aluminum foil pouches in a lab drawer until use.

The functional exposed groups of these C=C surfaces were converted into carboxyl groups -COOH by soaking the treated cover glass, as described above, for at least 15 min. in a solution of 25 mg KMnO₄, 750 mg NaIO₄ in 1 liter of H₂O, then rinsed 3 times in ultrapure de-ionized H₂O. The C =C surfaces were in some situations reacted with proteins. A volume of 300 µl of an aqueous solution (20 µg/ml) of protein (Protein A, streptavidin, etc.) was deposited on the surface and reacted with the functional vinyl group C=C. The surfaces were incubated, typically 1-2 hours at room temperature, then rinsed 3 times in a phosphate buffered saline (PBS). In this condition, the surfaces were clean but were then hydrophilic (they wet very well). The surfaces incubated in Protein A, were then converted to antibody (anti-DIG) surfaces, by incubating the Protein A surface, typically 1-2 hours at room temperature, with a solution of an antibody (20 µg/ml). It is believed that one should not provide too much Protein A or antibody to the surface, otherwise steric hindrance effects will detrimentally affect the reactivity of the surfaces.

### Example 3: Anchoring Native DNA to a C=C surface

Using a pipette tip with an enlarged opening (done by cutting the tip off) to prevent shearing of the DNA, a 5-10 µl aliquot of a 10⁷ molecules/µl solution (10⁻¹⁶ Moles) of λ-DNA was deposited onto a treated surface, freshly rinsed in running ultrapure water. For fluorescence based observations, the λ-DNA was freshly stained with YOYO-1 or other fluorophores. Then, a clean and freshly rinsed 18 mm round cover glass, typically untreated, was gently lowered down onto the drop of liquid, in such a manner to avoid shear forces breaking up the molecule. A very slow spreading of the drop was typically observed, often 10 seconds, before the moving contact line reached the edge of the round top cover glass.

There were at least two key control parameters, the time of incubation and the method of moving the meniscus. The solution was incubated anywhere from ten seconds to several hours, in either a dry or humid (100% RH) atmosphere, depending on the desired affect. Some samples were incubated 10-30 sec, and then mechanically sheared by pulling the top and bottom cover slips laterally across each other over a 5-10 second period, using tweezers to hold each outer edge of both surfaces. The time of incubation affects the percentage of DNA that binds because the binding process is diffusion limited and the probability of binding once in contact with the surface is constant. Thus, the longer the incubation time, the more binding. However, if one waits too long, overnight for example, then both ends of a given DNA molecule are likely to have bound, forming a U-shape or "loop," after passage of the meniscus, rather than straight linear segments. The utility of mechanical shearing, as described, is that is reduces the time necessary to wait to see the result, since shearing is fast (one minute at most) and evaporation for example, can take hours.

In a buffer of 50 mM MES, pH 5.5, the binding was observed homogeneously across well treated surfaces. In comparison, in a buffer of 10 mM Tris, 1 mM EDTA, pH 8.0, there is virtually no binding (less than 1 in 10⁶). This dependence can allow the control or activation of the surfaces relative to DNA binding by use of an intermediate pH.

### Example 4: Alignment and Detection of Anchored DNA by the Action of the Meniscus

Either by transferring the incubated sample to a dry environment and letting the solvent evaporate, or by shearing the sample mechanically, as described above, the passage of the meniscus extends the DNA molecules, anchored to the surface by one or both extremities, perpendicular to the meniscus (See, Figure 5). The force, suspected capillary in nature, on the DNA molecules (typically 10's of pN) is in fact sufficient to completely extend the molecule (that is larger than the elastic entropic force of the fluctuating Brownian coil), but is too weak to break the bond, perhaps covalent in nature, between the DNA and the silanated/treated surface. The DNA, if stained previously with a fluorophore (YOYO-1), is observed immediately and can be seen to be stretched, with a total length of 20-25 µm. The anchorage between the surface and the DNA is limited to the extremities, perhaps the 5' phosphate, and has also been observed with DNA of phage λ, of YAC, or of E. Coli (with a length greater than 450 µm). This preparation and fixation of the DNA, stretched, fluorescent and dried in the air is stable for several days, sometimes even weeks and months (if stored properly away from ambient light), and can be observed by epifluorescence microscopy, typically on an inverted microscope with a high magnification/high numerical aperture, oil immersion objective (a 100x/1.4 N. A. for example).

### Example 5: Specific Anchoring and Detection

After surface treatment, as described above, with a specific monoclonal antibody, one can control very precisely the surface specificity. Thus, the specificity of anti-DIG treated surfaces was tested vis-a-vis DNA hybridized with a complementary oligonucleotide to one of the cohesive or "sticky" ends and containing a digoxigene (DIG) versus non-hybridized DNA. In the first case, homogeneous extension was observed by the action of the meniscus, of those molecules anchored. In the second case, less than 10 DNA molecules were observed to be anchored in the entire sample. It is estimated that the specificity of the method of the present invention is better than 1:10⁶ (See, Subfigure 2A and Subfigure 2B) (See, Subfigures 2A and 2B).

### Example 6: Sensitive Detection

In order to determine the sensitivity of the detection method by the extension due to the passage of a meniscus, 2.5 µl aliquots of a solution of λ-DNA in 50 mM MES, pH 5.5 containing 10⁵, 10⁴, 10³ molecules were deposited on a surface. The binding was performed similarly to what was described above. The different surfaces were then observed under the epi-fluorescence microscope, in order to determine the density of the molecules anchored. For the lowest dilution (10³ molecules), one observed more than 10 DNA molecules (after searching 125 fields of view), which were extended by the action of the meniscus. This number is essentially limited by the large number of fields of view necessary to cover the entire sample (roughly 20,000). This renders manual search difficult, but can be advantageously accomplished either automatically by computer control of the stage and image processing, or with a low power objective. In conclusion, the sensitivity of the detection method of the present invention has already detected the presence of 1 in 100 DNA molecules, and is expected to have an even higher sensitivity (provide an even lower detection limit), to permit the detection of a single molecule.

### Example 7: Dependence of Stretching on Surface Treatment

The histogram of the measured lengths of extended λ-DNA molecules grafted to a silanated surface, after the action of a moving meniscus shows a well defined peak at 24 µm, whereas on a surface recovered with Protein A and then anti-DIG, the histogram of lengths of extended λ-DNA (cos end-labeled with a DIG molecule as described above), only shows a clear peak at 16 µm (often up to 18 µm). The stretching depends therefore on the surface treatment and surface preparation. The results may be shown as a histogram of the measured lengths of N fragments of λ-DNA molecules, and a typical image (inset), after (A) stretching by the passage of the meniscus on a hydrophobic surface (silane treated), or (B) one treated to become hydrophilic (with Protein A/anti-DIG). The figure demonstrates the dependence of the quality or degree of stretching upon the surface treatment and hydrophobicity (See, Figure 1, Bensimon et al., Phys. Rev. Lett. 74: 4754 (1995).

### Example 8: Homogeneous Stretching

In order to determine if the stretching is homogeneous (independent of the length of the molecule), a solution of multimers of λ-DNA was prepared by ligation using E. Coli T4 ligase. This solution was grafted on some silanated surfaces and then stretched by the action of the passing meniscus. The histogram of lengths of extended molecules shows 3 equidistant peaks corresponding to the lengths of monomers, dimers, and trimers. The stretching by the meniscus is therefore quite homogeneous. (Figure 4).

## Claims

1. A method for obtaining a surface S affording consistent, homogeneous and reproducible orientation, alignment, straightening or stretching of macromolecules, comprising :
cleaning a surface S of a substrate under conditions effective to obtain an organic molecule-free and dust-free surface S;
hydrating S; and
coating S with a desired material comprising exposed moieties which are capable of selectively binding macromolecules, the cleaning, hydrating and coating steps being conducted in a single chamber, and the surface S affording consistent, homogeneous and reproducible orientation of a macromolecule bound thereto.

2. The method of claim 1, including the further steps of
UV irradiating S in an oxidizing atmosphere ;
humidifying the atmosphere ; and
coating S with a polymer comprising an exposed reactive moiety.

3. A reproducible method of orienting, aligning, straightening or stretching a macromolecule on a surface, comprising
placing a macromolecule in contact with a surface S obtained by the method of claim 1 or 2 and a medium A, such that one site of the macromolecule is bound to S and a portion thereof is placed in A ; and
applying a random or non-random force to the macromolecule in contact with the medium A such as a gravitational, magnetic, electrophoretic, or temperature induced force under conditions effective to orient, align, straighten or stretch the macromolecule.

4. A reproducible method of orienting, aligning, straightening or stretching a macromolecule on a surface, comprising
placing a macromolecule in contact with a surface S obtained by the method of claim 1 or 2 and a medium A, such that one site of the macromolecule is bound to S and a portion thereof is placed in A ; and
mechanically translating the contents of A, relative to S under conditions effective to orient, align, straighten or stretch the macromolecule.

5. The method of claim 4, wherein said mechanically translating step being conducted by placing a level surface S' in contact with A, opposite S, and moving S or S' relative to the other.

6. A reproducible method of orienting, aligning, straightening or stretching a macromolecule on a surface, comprising
placing a macromolecule in contact with a surface S obtained by a method according to claim 1 or 2 and a medium A, such that one site of the macromolecule is bound to S and a portion thereof is placed in A ; and
mechanically translating the contents of A, relative to S under conditions effective to orient, align, straighten or stretch the macromolecule, said mechanically translating step being conducted by placing a level surface S' in contact with A, opposite S, and moving S or S' relative to the other.

7. The method according to anyone of claims 4 to 6, including the further step of adding a surfactant to A or to a medium having interfaces with A and S prior to the translating step.

8. The method according to anyone of claims 4 to 7, further comprising modifying S by anchoring onto S molecules which selectively bind a specific type of macromolecule, or portion thereof, prior to the translation step.

9. The method according to anyone of claims 3 to 7, applied to anyone of the following uses :
measuring a desired degree of orientation, alignment, straightening or stretching of a specific type of macromolecule bound to surface S,
the method including comparing, identifying, determining and/or separating the macromolecule or fragment thereof in relation with other molecules.

10. The method according to anyone of claims 3 to 7, applied to anyone of the following uses :
identifying a specific type of macromolecule present in a sample,
the method including comparing, identifying, determining and/or separating the macromolecule or fragment thereof in relation with other molecules.

11. The method according to anyone of claims 3 to 7, applied to anyone of the following uses :
determining the number of a specific type of macromolecule present in a known volume of a sample,
the method including comparing, identifying, determining and/or separating the macromolecule or fragment thereof in relation with other molecules.

12. The method according to anyone of claims 3 to 7, applied to anyone of the following uses :
separating a specific type of macromolecule from other types of macromolecules present in a sample,
the method including comparing, identifying, determining and/or separating the macromolecule or fragment thereof in relation with other molecules.

13. The method according to anyone of claims 3 to 7, applied to anyone of the following uses :
measuring the length of a specific type of macromolecule or fragment thereof,
the method including comparing, identifying, determining and/or separating the macromolecule or fragment thereof in relation with other molecules.

14. The method according to anyone of claims 3 to 13, wherein the binding of the specific type of macromolecule to the molecules is detected with a magnetic, coloured or fluorescent label.

15. The method according to anyone of claims 9 to 14, applied to measuring the length of a specific type of macromolecule or fragment thereof, the method including determining a desired distance by comparison to a determined or reference length.

16. A surface S obtained by a method according to claim 1, which is hydrophobic.

## Patentansprüche

1. Verfahren zum Erhalten einer Oberfläche S, die eine konsistente, homogene und reproduzierbare Orientierung, Ausrichtung, Geraderichtung oder Ausdehnung von Makromolekülen ermöglicht, wobei das Verfahren folgendes umfaßt:
Reinigen einer Oberfläche S eines Substrats unter Bedingungen, die effektiv sind, um eine von organischen Molekülen freie und staubfreie Oberfläche S zu erhalten,
Hydratisieren von S und
Beschichten von S mit einem gewünschten Material, welches freiliegende Reste umfaßt, die selektiv Makromoleküle binden können, wobei die Stufen der Reinigung, Hydratisierung und Beschichtung in einer einzelnen Kammer ausgeführt werden und die Oberfläche S eine konsistente, homogene und reproduzierbare Orientierung eines daran gebundenen Makromoleküls ermöglicht.

2. Verfahren nach Anspruch 1, welches die weiteren Stufen umfaßt, bei denen man
S in einer oxidierenden Atmosphäre mit UV-Licht bestrahlt,
die Atmosphäre befeuchtet und
S mit einem Polymer beschichtet, welches einen freiliegenden reaktiven Rest umfaßt.

3. Reproduzierbares Verfahren zum Orientieren, Ausrichten, Geraderichten oder Ausdehnen eines Makromoleküls auf einer Oberfläche, welches folgendes umfaßt:
inkontaktbringen eines Makromoleküls mit einer Oberfläche S, erhalten durch das Verfahren nach Anspruch 1 oder 2, und einem Medium A, so daß eine Stelle des Makromoleküls an S gebunden wird und ein Abschnitt davon in A angeordnet ist, und
Aufbringen einer zufälligen oder nicht-zufälligen Kraft, wie z.B. einer durch Gravitation, magnetisch, elektroforetisch oder durch Temperatur induzierten Kraft, auf das mit dem Medium A in Kontakt stehende Makromolekül unter Bedingungen, die effektiv sind, um das Makromolekül zu orientieren, auszurichten, geradezurichten oder auszudehnen.

4. Reproduzierbares Verfahren zum Orientieren, Ausrichten, Geraderichten oder Ausdehnen eines Makromoleküls auf einer Oberfläche, welches folgendes umfaßt:
Inkontaktbringen eines Makromoleküls mit einer Oberfläche S, erhalten durch das Verfahren nach Anspruch 1 oder 2, und einem Medium A, so daß eine Stelle des Makromoleküls an S gebunden wird und ein Abschnitt davon in A angeordnet ist, und
mechanisches Versetzen der Bestandteile von A relativ zu S unter Bedingungen, die effektiv sind, um das Makromolekül zu orientieren, auszurichten, geradezurichten oder auszudehnen.

5. Verfahren nach Anspruch 4, wobei die Stufe des mechanischen Versetzens dadurch ausgeführt wird, daß eine ebene Oberfläche S' gegenüber von S mit A in Kontakt gebracht wird und S oder S' jeweils relativ zueinander bewegt werden.

6. Reproduzierbares Verfahren zum Orientieren, Ausrichten, Geraderichten oder Ausdehnen eines Makromoleküls auf einer Oberfläche, welches folgendes umfaßt:
Inkontaktbringen eines Makromoleküls mit einer Oberfläche S erhalten durch ein Verfahren nach Anspruch 1 oder 2 und einem Medium A, so daß eine Stelle des Makromoleküls an S gebunden wird und ein Abschnitt davon in A angeordnet ist, und
mechanisches Versetzen der Bestandteile von A relativ zu S unter Bedingungen, die effektiv sind, um das Makromolekül zu orientieren, auszurichten, geradezurichten oder auszudehnen, wobei die Stufe des mechanischen Versetzens dadurch ausgeführt wird, daß eine ebene Oberfläche S' gegenüber von S mit A in Kontakt gebracht wird und S oder S' jeweils relativ zueinander bewegt werden.

7. Verfahren nach einem der Ansprüche 4 bis 6, welches die weitere Stufe umfaßt, bei der man vor der Stufe des Versetzens ein oberflächenaktives Mittel zu A oder zu einem Medium, welches Berührungsflächen mit A und S aufweist, zugibt.

8. Verfahren nach einem der Ansprüche 4 bis 7, welches vor der Stufe des Versetzens weiterhin das Modifizieren von S durch Verankern von Molekülen, die selektiv eine spezifische Art von Makromolekülen oder einen Teil davon binden, auf S umfaßt.

9. Verfahren nach einem der Ansprüche 3 bis 7, angewandt auf einen der nachstehenden Verwendungszwecke:
Messen eines gewünschten Ausmaßes der Orientierung, Ausrichtung, Geraderichtung oder Ausdehnung einer spezifischen Art von Makromolekülen, die an die Oberfläche S gebunden sind,
wobei das Verfahren das Vergleichen, Identifizieren, Bestimmen und/oder Trennen des Makromoleküls oder eines Fragments davon in Bezug auf andere Moleküle beinhaltet.

10. Verfahren nach einem der Ansprüche 3 bis 7, angewandt auf einen der nachstehenden Verwendungszwecke:
Identifizieren einer spezifischen Art von Makromolekülen, die in einer Probe vorliegen,
wobei das Verfahren das Vergleichen, Identifizieren, Bestimmen und/oder Trennen des Makromoleküls oder eines Fragments davon in Bezug auf andere Moleküle beinhaltet.

11. Verfahren nach einem der Ansprüche 3 bis 7, angewandt auf einen der nachstehenden Verwendungszwecke:
Bestimmen der Anzahl einer spezifischen Art von Makromolekülen, die in einer bekannten Menge einer Probe vorliegen,
wobei das Verfahren das Vergleichen, Identifizieren, Bestimmen und/oder Trennen des Makromoleküls oder eines Fragments davon in Bezug auf andere Moleküle beinhaltet.

12. Verfahren nach einem der Ansprüche 3 bis 7, angewandt auf einen der nachstehenden Verwendungszwecke:
Trennen einer spezifischen Art von Makromolekülen von anderen Arten von Makromolekülen, die in einer Probe vorliegen,
wobei das Verfahren das Vergleichen, Identifizieren, Bestimmen und/oder Trennen des Makromoleküls oder eines Fragments davon in Bezug auf andere Moleküle beinhaltet.

13. Verfahren nach einem der Ansprüche 3 bis 7, angewandt auf einen der nachstehenden Verwendungszwecke:
Messen der Länge einer spezifischen Art von Makromolekülen oder eines Fragments davon,
wobei das Verfahren das Vergleichen, Identifizieren, Bestimmen und/oder Trennen des Makromoleküls oder eines Fragments davon in Bezug auf andere Moleküle beinhaltet.

14. Verfahren nach einem der Ansprüche 3 bis 13, wobei die Bindung der spezifischen Art von Makromolekülen an die Moleküle mittels einer magnetischen, farbigen oder fluoreszenten Markierung detektiert wird.

15. Verfahren nach einem der Ansprüche 9 bis 14, angewandt auf das Messen der Länge einer spezifischen Art von Makromolekülen oder eines Fragments davon, wobei das Verfahren das Bestimmen eines gewünschten Abstands durch Vergleichen mit einer bestimmten Länge oder Referenzlänge beinhaltet.

16. Oberfläche S, erhalten durch ein Verfahren nach Anspruch 1, welche hydrophob ist.

## Revendications

1. Procédé d'obtention d'une surface S qui assure des orientation, alignement, redressement ou allongement reproductibles, homogènes et fiables de macromolécules comprenant :
le nettoyage d'une surface S d'un substrat dans des conditions assurant l'obtention efficace d'une surface S dépourvue de molécules organiques et de poussières,
l'hydratation de la surface S, et
le revêtement de la surface S d'une matière voulue comprenant des motifs exposés qui sont capables d'assurer la liaison sélective de macromolécules, les étapes de nettoyage, d'hydratation et de revêtement étant exécutées dans une chambre unique, et la surface S assurant une orientation reproductible, homogène et fiable d'une macromolécule qui lui est liée.

2. Procédé selon la revendication 1, comprenant les étapes supplémentaires suivantes :
l'irradiation ultraviolette de la surface S dans une atmosphère oxydante,
l'humidification de l'atmosphère, et
le revêtement de la surface S d'un polymère comprenant un motif réactif exposé.

3. Procédé reproductible d'orientation, d'alignement, de redressement ou d'allongement d'une molécule sur une surface, comprenant :
la disposition d'une macromolécule au contact d'une surface S obtenue par le procédé selon la revendication 1 ou 2, et d'un milieu A, afin qu'un site de la macromolécule soit lié à la surface S et qu'une de ses parties soit placée dans le milieu A, et
l'application d'une force, aléatoire ou non, à la macromolécule au contact du milieu A, telle qu'une force gravitationnelle, magnétique, d'électrophorèse ou induite par la température, dans des conditions qui assurent efficacement l'orientation, l'alignement, le redressement ou l'allongement de la macromolécule.

4. Procédé reproductible d'orientation, d'alignement, de redressement ou d'allongement d'une macromolécule sur une surface, comprenant :
la disposition d'une macromolécule au contact d'une surface S obtenue par le procédé selon la revendication 1 ou 2 et d'un milieu d'une manière telle qu'un site de la macromolécule est lié à la surface S et une partie de celle-ci est placée dans le milieu A, et
le déplacement mécanique en translation du contenu du milieu A par rapport à la surface S dans des conditions qui assurent efficacement l'orientation, l'alignement, le redressement ou l'allongement de la macromolécule.

5. Procédé selon la revendication 4, dans lequel l'étape de transmission mécanique est exécutée par disposition d'une surface de niveau S' au contact du milieu A, en regard de la surface S, et le déplacement de la surface S ou S' par rapport à l'autre surface.

6. Procédé reproductible d'orientation, d'alignement, de redressement ou d'allongement d'une macromolécule sur une surface, comprenant :
la disposition d'une macromolécule au contact d'une surface S obtenue par un procédé selon la revendication 1 ou 2 et d'un milieu A, de manière qu'un site de la macromolécule soit lié à la surface S et qu'une de ses parties soit placée dans le milieu A, et
le déplacement mécanique en translation du contenu du milieu A par rapport à la surface S dans des conditions qui assurent efficacement l'orientation, l'alignement, le redressement ou l'allongement de la macromolécule, l'étape de translation mécanique étant exécutée par disposition d'une surface de niveau S' au contact du milieu A, en face de la surface S, et le déplacement de la surface S ou S' par rapport à l'autre surface.

7. Procédé selon l'une quelconque des revendications 4 à 6, comprenant une étape supplémentaire d'addition d'un agent tensioactif au milieu A ou à un milieu ayant des interfaces avec le milieu A et la surface S avant l'étape de déplacement en translation.

8. Procédé selon l'une quelconque des revendications 4 à 7, comprenant en outre la modification de la surface S par ancrage à la surface S de molécules qui fixent sélectivement un type spécifique de macromolécule ou une partie d'une telle macromolécule, avant l'étape de déplacement en translation.

9. Procédé selon l'une quelconque des revendications 3 à 7, appliqué à l'une quelconque des applications suivantes :
la mesure d'un degré d'orientation, d'alignement, de redressement ou d'allongement d'un type spécifique de molécule liée à la surface S,
le procédé comprenant la comparaison, l'identification, la détermination et/ou la séparation de la macromolécule ou d'un de ses fragments d'autres molécules.

10. Procédé selon l'une quelconque des revendications 3 à 7, appliqué à l'une quelconque des applications suivantes :
l'identification d'un type spécifique de macromolécule présent dans un échantillon,
le procédé comprenant la comparaison, l'identification, la détermination et/ou la séparation de la macromolécule ou d'un de ses fragments d'autres molécules.

11. Procédé selon l'une quelconque des revendications 3 à 7, appliqué à l'une quelconque des applications suivantes :
la détermination du nombre de macromolécules d'un type spécifique présentes dans un volume donné d'échantillon,
le procédé comprenant la comparaison, l'identification, la détermination et/ou la séparation de la macromolécule ou d'un de ses fragments d'autres molécules.

12. Procédé selon l'une quelconque des revendications 3 à 7, appliqué à l'une quelconque des applications suivantes :
la séparation d'un type spécifique de macromolécule d'autres types de macromolécules présentes dans un échantillon,
le procédé comprenant la comparaison, l'identification, la détermination et/ou la séparation de la macromolécule ou d'un de ses fragments d'autres molécules.

13. Procédé selon l'une quelconque des revendications 3 à 7, appliqué à l'une quelconque des applications suivantes :
la mesure de la longueur d'un type spécifique de macromolécule ou d'un de ses fragments,
le procédé comprenant la comparaison, l'identification, la détermination et/ou la séparation de la macromolécule ou d'un de ses fragments d'autres molécules.

14. Procédé selon l'une quelconque des revendications 3 à 13, dans lequel la liaison du type spécifique de macromolécule aux molécules est détectée par un marqueur magnétique, coloré ou fluorescent.

15. Procédé selon l'une quelconque des revendications 9 à 14, appliqué à la mesure de la longueur d'un type spécifique de macromolécule ou d'un de ses fragments, le procédé comprenant la détermination d'une distance voulue par comparaison à une longueur déterminée ou de référence.

16. Surface S obtenue par un procédé selon la revendication 1, qui est hydrophobe.
